# EUROPÄISCHE PATENTSCHRIFT

(11) **EP 1 100 457 B1**
(45) Veröffentlichungstag und Bekanntmachung des Hinweises auf die Patenterteilung: **06.10.2004**
(21) Anmeldenummer: 99941453.5
(22) Anmeldetag: 22.07.1999
(51) Int. Cl.: A61K 7/48

(54) **VERWENDUNG VON ECTOIN ODER ECTOIN-DERIVATEN IN KOSMETISCHEN FORMULIERUNGEN**
USE OF ECTOINE OR ECTOINE DERIVATIVES IN COSMETIC FORMULATIONS
UTILISATION D'ECTOINE OU DE DERIVES D'ECTOINE DANS DES FORMULATIONS COSMETIQUES

(30) Priorität: 01.08.1998 DE 19834817; 17.03.1999 DE 19911775
(43) Veröffentlichungstag der Anmeldung: 23.05.2001
(73) Patentinhaber: MERCK PATENT GmbH, 64293 Darmstadt (DE)
(72) Erfinder: BÜNGER, Joachim, D-64823 Gro -Umstadt (DE); DRILLER, Hans-Jürgen, D-64823 Gro -Umstadt (DE); MARTIN, Roland, D-69469 Weinheim (DE)
(86) Internationale Anmeldenummer: PCT/EP1999/005239
(87) Internationale Veröffentlichungsnummer: WO 2000/007560

(56) Entgegenhaltungen:
- DE-A- 4 342 560
- J. BÜNGER: "Neue Wirkstoffklasse schützt und pflegt die Haut" PARFÜMERIE UND KOSMETIK, Bd. 79, Nr. 11, 1998, Seiten 32,34-35, XP000853882
- PAPE W.J.W.; HOPPE U.: 'Standardization of an in vitro red Blood Cell Test for Evaluating the Acute Cytotoxic Potential of Tensides' ARZNEIMITTEL-FORSCHUNG/DRUG RESEARCH Bd. 40, Nr. 4, 1990, AULENDORF, Seiten 498 - 502

## Beschreibung

Die Erfindung betrifft die Verwendung von einer oder mehreren Verbindungen ausgewählt aus den Verbindungen der Formeln Ia und Ib 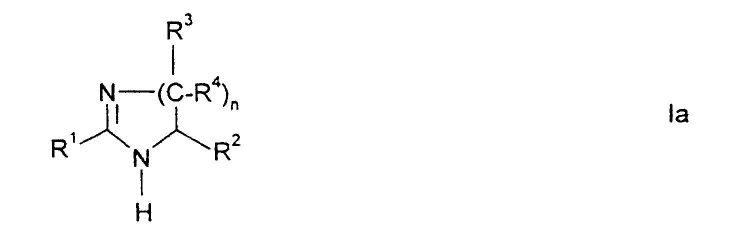 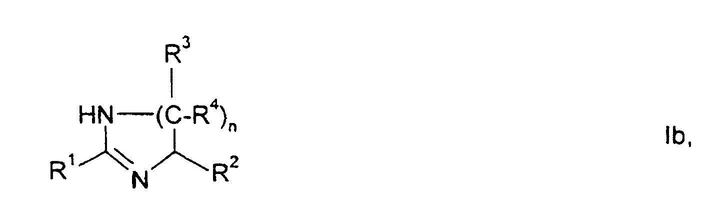 den physiologisch verträglichen Salzen der Verbindungen der Formeln la und Ib, und den stereoisomeren Formen der Verbindungen der Formeln la und Ib, wobei
- R¹: H oder Alkyl,
- R²: H, COOH, COO-Alkyl oder CO-NH-R⁵,
- R³ und R⁴: jeweils unabhängig voneinander H oder OH,
- n: 1, 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten, zur Herstellung einer kosmetischen Formulierung
- zum Schutz von Zellen, Proteinen und/oder Biomembranen der menschlichen Haut, gegen die Schädigenden Wirkung von Tensiden,
- zum Schutz der Mikroflora der menschlichen Haut gegen die Schädigende Wirkung von Tensiden und/oder
- zur Stabilisierung der Hautbarriere gegen die Schädigende Wirkung von Tensiden.

Die gesunde menschliche Haut wird an ihrer Oberfläche, dem Stratum corneum, von einer großen Anzahl kommensalisch lebender Mikroorganismen kolonisiert. Aus der großen Vielfalt dieser Mikroorganismen leben nur wenige ständig auf der Haut und bilden so die residente Hautflora. Die Hauptvertreter der residenten Flora auf der menschlichen Haut sind Staphylococcen, Micrococcen, coryneforme Bakterien und Pityrosporen. Diese leben in kleinen Kolonien auf der Oberfläche des Stratum corneum und in der äußeren Epidermis. Eine zweite Gruppe von Mikroorganismen, die sich vorübergehend von außen, insbesondere auf exponierten Hautbereichen, ansiedelt, wird als transiente Flora bezeichnet und kann sich auf der gesunden Haut, deren Mikromilieu stark durch die residente Mikroflora bestimmt wird, nicht dauerhaft ansiedeln. In unterschiedlichen Körperregionen variiert die Zusammensetzung der Hautflora in Abhängigkeit vom Mikromilieu der Haut. Die Dichte der Mikroorganismen paßt sich dem jeweiligen Hautmilieu an, so daß die Ökologie dieser Körperregionen nicht durch eine übermäßige Besiedlung durch Mikroorganismen aus dem Gleichgewicht gebracht wird. Im Vergleich zum Normalzustand der Haut nimmt die Anzahl der Mikroorganismen bei trockener Haut ab, während die Anzahl der Mikroorganismen bei feuchter Haut, z.B. durch entzündliche Veränderungen bei einem Ekzem, bis um das 1000-fache zunimmt.

Die Haut ist als Grenzschicht und Oberfläche des menschlichen Körpers einer Vielzahl externer Streßfaktoren ausgesetzt. Die Human-Haut ist ein Organ, das mit verschiedenartig spezialisierten Zelltypen - den Keratinozyten, Melanozyten, Langerhans-Zellen, Merkel-Zellen und eingelagerten Sinneszellen - den Körper vor äußeren Einflüssen schützt. Hierbei ist zwischen äußeren physikalischen, chemischen und biologischen Einflüssen auf die menschliche Haut zu unterscheiden. Zu den äußeren physikalischen Einflüssen sind thermische und mechanische Einflüsse sowie die Einwirkung von Strahlen zu zählen. Unter den äußeren chemischen Einflüssen sind insbesondere die Einwirkung von Toxinen und Allergenen zu verstehen. Die äußeren biologischen Einflüsse umfassen die Einwirkung fremder Organismen und deren Stoffwechselprodukte.

Die Oberfläche der menschlichen Haut wird von einem Fettfilm bedeckt, der, je nach den gegebenen Verhältnissen, als eine Öl-in-Wasser- oder eine Wasser-in-Öl-Emulsion anzusehen ist und zahlreiche Wirkstoffe, wie z.B. Enzyme und Vitamine, z.B. Vitamin D, enthält. Dieser Fettfilm, der aus den von Talgdrüsen und Keratinozyten abgegebenen Lipiden gebildet wurde, bewahrt die Feuchtigkeit der Haut und schützt den Körper als Hautbarriere vor ungünstigen Umweltfaktoren. Dieses empfindliche Gleichgewicht der Hautbarriere wird durch externe oder interne Faktoren gestört.

Die Mikroorganismen der menschlichen Haut sind verschiedenen Streßfaktoren ausgesetzt. Beispielsweise können sie durch Austrocknung oder durch hohe Salzkonzentrationen auf der Hautoberfläche, z.B. nach dem Schwitzen, geschädigt werden, was eine Schädigung der Hautbarriere zur Folge haben kann. Einige dieser Mikroorganismen - Staphylococcen, Micrococcen, Corynebakterien und Brevibakterien - besitzen jedoch üblicherweise die Fähigkeit, Kompatible Solute zu bilden, um sich gegen Austrocknung oder hohe Salzkonzentration zu schützen und tragen somit zur Ausbildung einer intakten Hautbarriere bei. Die Kompatiblen Solute, die auch als Streßschutzstoffe bezeichnet werden, sind niedermolekulare Substanzen im Cytoplasma.

Bisher wurde beispielsweise der Versuch unternommen, die Pflege oder den Schutz der menschlichen Haut durch hydrophile Substanzen, die selbst Wasser binden, zu bewirken (E.A. Galinski, Experientia 49 (1993) 487-496). Diese hydrophilen Substanzen binden jedoch Wassermoleküle des Hydratationswassers ebenso wie freie Wassermoleküle. Dadurch kommt es zwar zu einer Bindung von Wassermolekülen, nicht jedoch beispielsweise zu einem Schutz der Hydrathüllen von Zellen, Proteinen und Zellmembranen.

Es bestand daher die Aufgabe, kosmetische Formulierungen zur Verfügung zu stellen, deren Anwendung die obengenannten Hautprobleme beseitigen oder zumindest mindern und insbesondere
- zum Schutz von Zellen, Proteinen, und/oder Biomembranen der menschlichen Haut gegen die Schädigende Wirkung von Tensiden
- zum Schutz der Mikroflora der menschlichen Haut gegen die Schädigende Wirkung von Tensiden
und/oder
- zur Stabilisierung der Hautbarriere gegen die Schädigende Wirkung von Tensiden
geeignet sind.

Überraschend wurde nun gefunden, daß diese Aufgabe durch die Verwendung von einer oder mehreren Verbindungen ausgewählt aus den Verbindungen der Formeln la und Ib 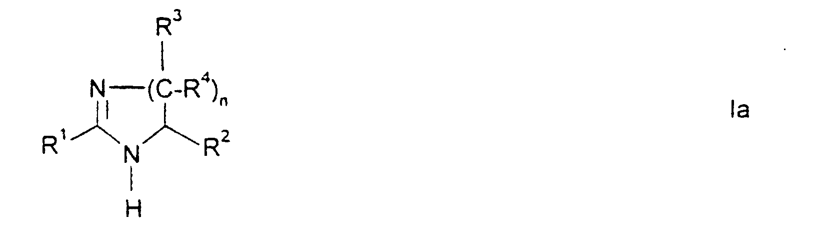 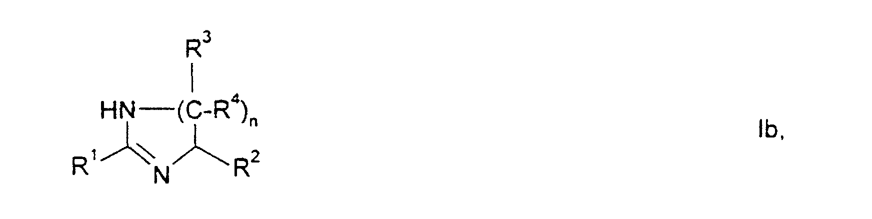 den physiologisch verträglichen Salzen der Verbindungen der Formeln la und Ib, und den stereoisomeren Formen der Verbindungen der Formeln la und Ib, wobei
- R¹: H oder Alkyl,
- R²: H, COOH, COO-Alkyl oder CO-NH-R⁵,
- R³ und R⁴: jeweils unabhängig voneinander H oder OH,
- n: 1, 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten,

in kosmetischen Formulierungen gelöst wird.

Im Rahmen der vorliegenden Erfindung werden alle vor- und nachstehenden Verbindungen ausgewählt aus den Verbindungen der Formeln Ia und Ib, den physiologisch verträglichen Salzen der Verbindungen der Formeln la und Ib, und den stereoisomeren Formen der Verbindungen der Formeln la und Ib als "Ectoin oder Ectoin-Derivate" bezeichnet.

Ectoinhaltige kosmetische Formulierungen schützen Zellen, Proteine, Enzyme, Vitamine, DNA, Zell- und Biomembranen der Haut vor den Schäden durch Austrocknung und Wasserentzug. Durch die Hydratationswirkung des Ectoins wird das Wassergleichgewicht des Stratum corneums sowie die Hautbarriere stabilisiert. Ectoin beugt einer trockenen und schuppigen Haut vor.

Ectoinhaltige kosmetische Formulierungen schützen zudem die für eine intakte Hautbarriere wichtige Mikroflora der Haut gegen Streß durch Austrocknung und hohe Ionenkonzentration nach dem Schwitzen. Die Stabilisierung der residenten Hautflora durch Ectoin oder seine Derivate ist eine wichtige Voraussetzung für das Gleichgewicht des Mikromilieus der Haut und die Ausbildung einer intakten Hautbarriere.

Bei Ectoin und den Ectoin-Derivaten handelt es sich um niedermolekulare. cyclische Aminosäurederivate, die aus verschiedenen halophilen Mikroorganismen gewonnen werden können. Sowohl Ectoin als auch Hydroxyectoin besitzen den Vorteil, daß sie nicht mit dem Zellstoffwechsel reagieren.

in der DE 43 42 560 wird die Verwendung von Ectoin und Ectoin-Derivaten als Feuchtigkeitsspender in Kosmetikprodukten beschrieben.

Die Verbindungen ausgewählt aus den Verbindungen der Formeln la und Ib, den physiologisch verträglichen Salzen der Verbindungen der Formeln Ia und Ib und den stereoisomeren Formen der Verbindungen der Formeln Ia und Ib können in den kosmetischen Zubereitungen als optische Isomere, Diastereomere, Racemate, Zwitterionen, Kationen oder als Gemisch derselben vorliegen. Unter den Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib, sind diejenigen Verbindungen bevorzugt, worin R¹ H oder CH₃, R² H oder COOH, R³ und R⁴ jeweils unabhängig voneinander H oder OH und n 2 bedeuten. Unter den Verbindungen ausgewählt aus den Verbindungen der Formeln Ia und Ib, den physiologisch verträglichen Salzen der Verbindungen der Formeln la und Ib und den stereoisomeren Formen der Verbindungen der Formeln la und Ib sind die Verbindungen (S)-1,4,5,6-Tetrahydro-2-methyl-4-pyrimidincarbonsäure (Ectoin) und (S,S)-1,4,5,6-Tetrahydro-5-hydroxy-2-methyl-4-pyrimidincarbonsäure (Hydroxyectoin) insbesondere bevorzugt.

Unter dem Begriff "Aminosäure" werden die stereoisomeren Formen, z.B. D- und L-Formen, folgender Verbindungen verstanden: Alanin, β-Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin, γ-Aminobutyrat, Nε-Acetyllysin, Nδ-Acetylornithin, Nγ-Acetyldiaminobutyrat und Nα-Acetyldiaminobutyrat. L-Aminosäuren sind bevorzugt.

Aminosäurereste leiten sich von den entsprechenden Aminosäuren ab.

Die Reste folgender Aminosäuren sind bevorzugt: Alanin, β-Alanin, Asparagin, Asparaginsäure, Glutamin, Glutaminsäure, Glycin, Serin, Threonin, Valin, γ-Aminobutyrat, Nε-Acetyllysin, Nδ-Acetylornithin, Nγ-Acetyldiaminobutyrat und Nα-Acetyldiaminobutyrat.

Die Di- und Tripeptidreste sind ihrer chemischen Natur nach Säureamide und zerfallen bei der Hydrolyse in 2 oder 3 Aminosäuren. Die Aminosäuren in den Di- und Tripeptidresten sind durch Amidbindungen miteinander verbunden. Bevorzugte Di- und Tripetidreste sind aus den bevorzugten Aminosäuren aufgebaut.

Die Alkylgruppen umfassen die Methylgruppe CH₃, die Ethylgruppe C₂H₅, die Propylgruppen CH₂CH₂CH₃ und CH(CH₃)₂ sowie die Butylgruppen CH₂CH₂CH₂CH₃, H₃CCHCH₂CH₃, CH₂CH(CH₃)₂ und C(CH₃)₃. Die bevorzugte Alkylgruppe ist die Methylgruppe.

Bevorzugte physiologisch verträgliche Salze der Verbindungen der Formeln la und Ib sind beispielsweise Alkali-, Erdalkali- oder Ammoniumsalze, wie Na-, K-, Mg- oder Ca-Salze, sowie Salze abgeleitet von den organischen Basen Triethylamin oder Tris-(2-hydroxy-ethyl)-amin. Weitere bevorzugte physiologisch verträgliche Salze der Verbindungen der Formeln Ia und Ib ergeben sich durch Umsetzung mit anorganischen Säuren wie Salzsäure, Schwefelsäure und Phosphorsäure oder mit organischen Carbon- oder Sulfonsäuren wie Essigsäure, Citronensäure, Benzoesäure, Maleinsäure, Fumarsäure, Weinsäure und p-Toluolsulfonsäure.

Verbindungen der Formeln la und Ib, in denen basische und saure Gruppen wie Carboxyl- oder Aminogruppen in gleicher Zahl vorliegen, bilden innere Salze.

Die Herstellung der Verbindungen der Formel la und Ib ist in der Literatur beschrieben (DE 43 42 560). (S)-1,4,5,6-Tetrahydro-2-methyl-4-pyrimidincarbonsäure oder (S,S)-1,4,5,6-Tetrahydro-5-hydroxy-2-methyl-4-pyrimidincarbonsäure können auch mikrobiologisch gewonnen werden (Severin et al., J. Gen. Microb. 138 (1992) 1629-1638).

Die Herstellung der kosmetischen Formulierung erfolgt, indem eine oder mehrere Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib gegebenenfalls mit Hilfs- und/oder Trägerstoffen in eine geeignete Formulierungsform gebracht werden. Die Hilfs- und Trägerstoffe stammen aus der Gruppe der Trägermittel, Konservierüngsstoffe und anderer üblicher Hilfsstoffe.

Die kosmetischen Formulierungen auf der Grundlage einer oder mehrerer Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib werden äußerlich angewendet.

Als Anwendungsform seien z.B. genannt: Lösungen, Suspensionen, Emulsionen, Pasten, Salben, Gele, Cremes, Lotionen, Puder, Seifen, tensidhaltige Reinigungspräparate, Öle und Sprays. Zusätzlich zu einer oder mehreren Verbindungen ausgewählt aus den Verbindungen der Formeln Ia und Ib, den physiologisch verträglichen Salzen der Verbindungen der Formeln la und Ib und den stereoisomeren Formen der Verbindungen der Formeln Ia und Ib werden der Formulierung beliebige übliche Trägerstoffe, Hilfsstoffe und gegebenenfalls weitere Wirkstoffe zugesetzt.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können neben einer oder mehrerer Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Der Anteil der Verbindungen ausgewählt aus den Verbindungen der Formeln la und Ib, den physiologisch verträglichen Salzen der Verbindungen der Formeln la und Ib und den stereoisomeren Formen der Verbindungen der Formeln Ia und Ib in der kosmetischen Formulierung beträgt vorzugsweise von 0,0001 bis 50 Gew.%, besonders bevorzugt von 0,001 bis 10 Gew.% bezogen auf die gesamte kosmetische Formulierung.

Der Schutz der Haut vor Austrocknung kann beispielsweise in vivo nachgewiesen werden, z.B. durch bekannte Nachweismethoden wie TEWL (transepidermal water loss), Corneometrie (zur Bestimmung der Hautfeuchtigkeit), Mikrotopographie (zur Bestimmung der Hautrauhigkeit) oder SELS (surface evaluation of living skin).

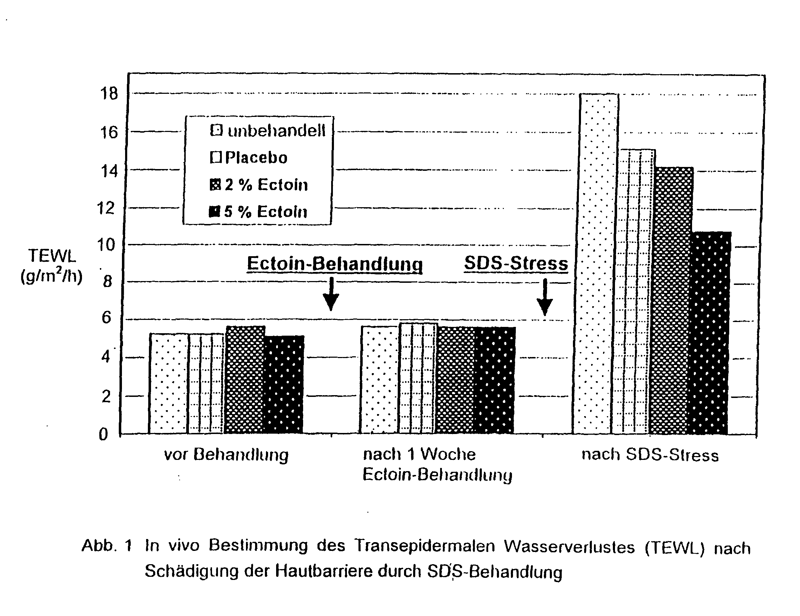

Ectoin-haltige Formulierungen können beispielsweise die Hautbarriere gegen die schädigende Wirkung von Natriumdodecylsulfat (SDS) schützen. Durch die Anwendung einer kosmetischen Ectoin-haltigen Emulsion kann der transepidermale Wasserverlust z.B. bis zu 40 % deutlich reduziert werden (Abb. 1). Eine mit einer Ectoin-haltigen kosmetischen Formulierung vorbehandelte Haut ist unempfindlicher gegenüber einer Schädigung der Hautbarriere durch das Tensid SDS. Durch die Anwendung einer Ectoin-haltigen Emulsion ist die Haut besser gegen eine Tensidschädigung der Haut und den damit einhergehenden Wasserverlust geschützt.

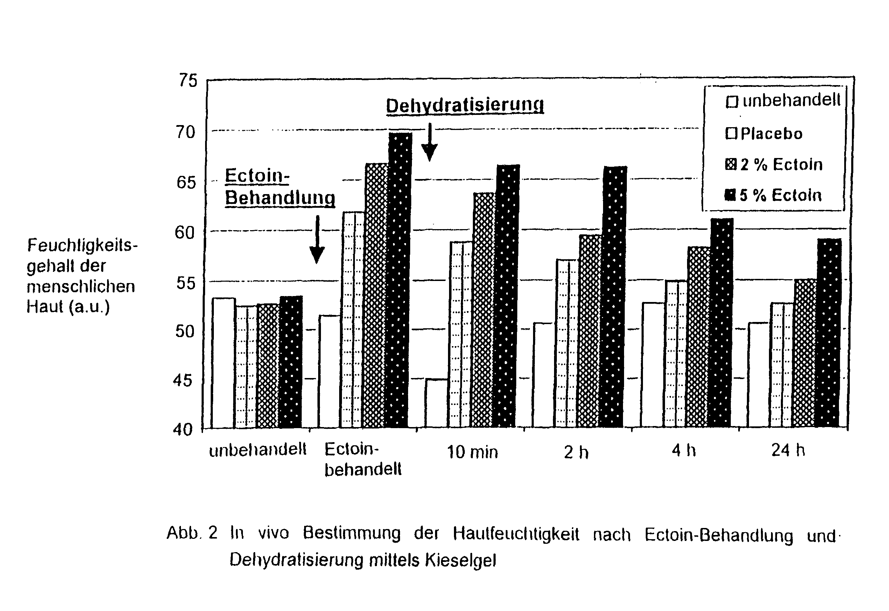

Ein wichtiges Ziel der Kosmetik ist nach wie vor ein Schutz der Haut gegen Stressfaktoren, die zum Austrocknen der Haut führen. Insbesondere trockene Luft während kalter oder sehr warmer Wetterlagen führt zu einem starken Wasserverlust der Haut. Ectoin schützt z.B. aus einer kosmetischen O/W-Emulsion heraus vor Austrocknung (Abb. 2). Zusätzlich zu dem Schutz gegen Austrocknung führen Ectoin-haltige kosmetische Formulierungen zu einer deutlich besseren Hautfeuchtigkeit als eine entsprechende Grundformulierung ohne Ectoin (Placebo), die aber bereits 3 % Glycerin enthält. Desweiteren bewirken Ectoin-haltige kosmetische Formulierungen auch nach 24 Stunden noch eine deutlich höhere Hautfeuchtigkeit im Vergleich zum unbehandelten oder nur mit dem Placebo behandelten Hautareal. Ectoin-haltige kosmetische Formulierungen schützen die Haut vor einer schnellen Austrocknung selbst gegen stark hygroskopisches Kieselgel, welches direkt auf die Haut aufgetragen ist. Die Feuchtigkeit der Haut kann durch die topische Anwendung von Ectoin-haltigen kosmetischen Formulierungen über einen längeren Zeitraum gegen Austrocknung geschützt werden. Ectoin-haltige kosmetische Formulierungen sind somit für eine Prophylaxe gegen trockene Haut gut geeignet.

Die Stabilisierung der Biomembranen kann z.B. in vitro nachgewiesen werden. Hierbei wird ausgenutzt, daß Propidiumiodid bei intakter Membran der Hautzellen nicht in die Zellen aufgenommen wird und tote Zellen oder Zellen mit einer geschädigten Membran für Propidiumiodid permeabel sind und durch die Propidiumiodid-Aufnahme einer Rotfärbung unterliegen.

Durch Vergleich von Zellkulturen, die vor der Schädigung, beispielsweise durch DMSO-Zugabe, mit Ectoin vorbehandelt wurden und von nicht vorbehandelten Zellen, kann nach anschließender Propidiumiodid-Behandlung die Wirkung des Ectoins oder seiner Derivate auf die Stabilisierung der Biomembranen festgestellt werden.

Zur Bestimmung der Zellmembran- und Protein-schädigenden Wirkung von Tensiden kann z.B. der RBC-Test verwendet werden. Hierzu werden die Erythrozyten z.B. mit Natriumdodecylsulfat (SDS) inkubiert, beispielsweise für eine Dauer von 10 Minuten. SDS destabilisiert die Membran unbehandelter Zellen so, daß die Zellen teilweise lysiert werden und ihre Inhaltsstoffe wie das Hämoglobin frei geben. Das bei der Zellwandschädigung freigesetzte Hämoglobin dient als Indikator für die spektrophotometrische Bestimmung der Membranschädigung durch SDS. Anhand des freigesetzten Hämoglobins kann die Anzahl der zerstörten Erythrozyten bestimmt werden.

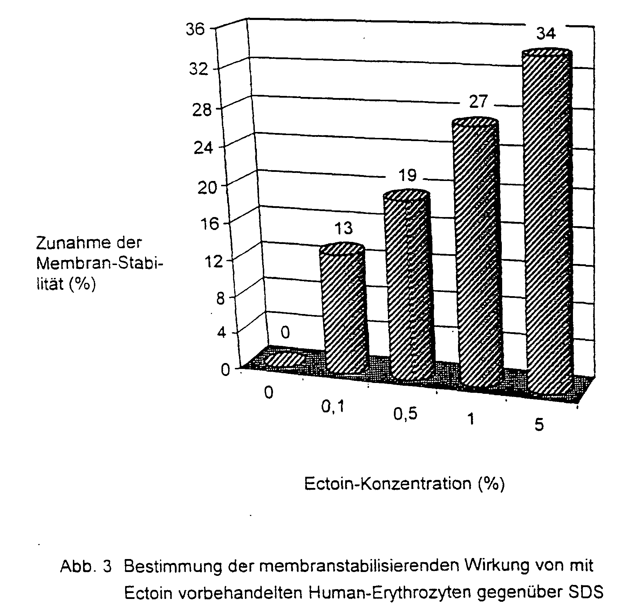

Ectoin schützt die Zellen gegen eine Schädigung durch SDS (Abb. 3). Die mit Ectoin vorbehandelten Erythrozyten sind gegenüber einer Membranschädigung durch SDS resistenter als unbehandelte Zellen. Je höher die Ectoin-Konzentration, desto größer ist der Schutzeffekt gegen eine Membranschädigung.

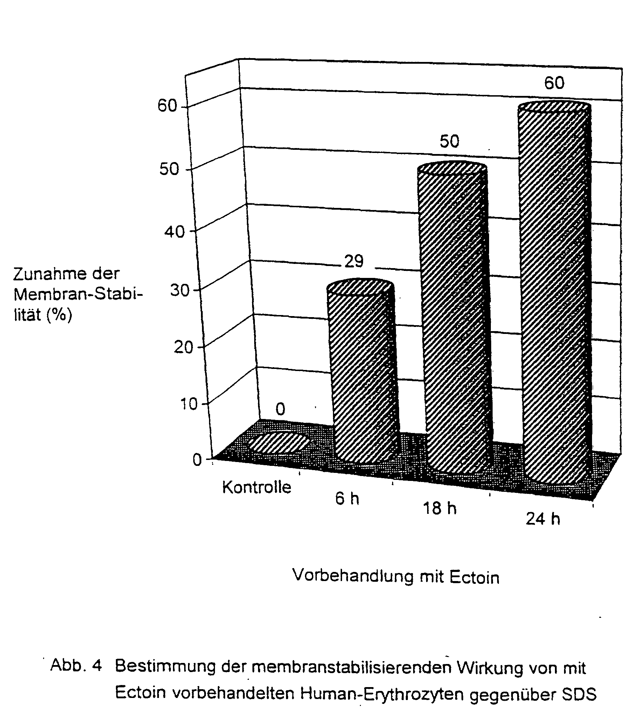

Je länger die Zellen mit Ectoin vorbehandelt werden, desto größer ist der Schutzeffekt gegen eine Membranschädigung (Abb. 4). Die Stabilisierung der Zellmembranen ist sowohl von der Ectoin-Konzentration abhängig, als auch von der Dauer der Ectoin-Vorbehandlung. Je höher die Ectoin-Konzentration und je länger die Einwirkzeit auf die Erythrozyten ist, desto stärker werden die Zellmembranen geschützt.

Der Nachweis der Stabilisierung der residenten Mikroflora kann beispielsweise in vivo erfolgen. Nach Ectoin-Behandlung bestimmter Hautbereiche. beispielsweise der Unterarme, wird die Haut z.B. Trocken- und/oder Hitzestreß in einer Klimakammer ausgesetzt. Anschließend werden die Bakterien von den Unterarmen isoliert und eine "Lebend-Zellzahlbestimmung" mittels Vitalfärbung sowie eine Wachstumskurve zur Bestimmung der Kinetik, beispielsweise durch das Ausplattieren der Bakterien auf Kulturplatten (Plattenverfahren) oder durch das Impedanzverfahren mittels Leitfähigkeitsmessungen, durchgeführt. Ein Vergleich dieser Ergebnisse mit denen für nicht vorbehandelte Hautbereiche liefert einen Nachweis der Wirkung des Ectoins oder seiner Derivate auf die Stabilisierung der residenten Mikroflora.

Alle Verbindungen oder Komponenten, die in den kosmetischen Formulierungen verwendet werden können, sind entweder bekannt und käuflich erwerbbar oder können nach bekannten Methoden synthetisiert werden.

Die folgenden Beispiele dienen zur Verdeutlichung der Erfindung und sind keinesfalls als Limitierung aufzufassen. Alle %-Angaben sind Gewichtsprozent.

Die INCI-Namen verwendeter Rohstoffe sind wie folgt (die INCI-Namen werden defintionsgemäß in Englischer Sprache angegeben):

| Rohstoff | INCl-Name |
|---|---|
| Mandelöl | Sweet Almond Oil (Prunus Dulcis) |
| Eutanol G | Octyldodecanol |
| Luvitol EHO | Cetearyl Octanoate |
| Oxynex K flüssig | PEG-8, Tocopherol, Ascorbyl Palmitate, Ascorbic Acid, Citric Acid |
| | |
| Panthenol | Panthenol |
| Karion F flüssig | Sorbitol |
| Sepigel 305 | Polyacrylamide, C13-14 Isoparaffin, Laureth-7 |
| | |
| Paraffin, dünnflüssig | Mineral Oil (Paraffinum Liquidum) |
| Mirasil CM 5 | Cyclomethicone |
| Arlacel 165 | Glyceryl Stearate, PEG-100 Stearate |
| Germaben II | Propylene Glycol, Diazolidinyl urea, Methylparaben, Propylparaben |
| | |
| Parfüm Bianca | Parfum |
| Abil WE 09 | Polyglyceryl-4 Isostearate, Cetyl Dimethicone Copolyol, Hexyl Laurate |
| | |
| Jojobaöl | Jojoba Oil (Buxus Chinensis) |
| Cetiol V | Decyl Oleate |
| Prisorine IPIS 2021 | Isopropyl Isostearate |
| Ricinusöl | Castor Oil (Ricinus Communis) |
| Lunacera M | Cera Microcristallina |
| Miglyol 812 Neutralöl | Caprylic/Capric Triglyceride |
| Eusolex T-2000 | Titanium Dioxide, Alumina, Simethicone |

### Beispiel 1

Aus folgenden Komponenten wird ein erfindungsgemäßes Hautpflegegel (O/W) enthaltend Ectoin hergestellt:

| | | | | Gew.-% |
|---|---|---|---|---|
| A | Mandelöl | | (2) | 8.0 |
| | Eutanol G | | (3) | 2.0 |
| | Luvitol EHO | | (4) | 6.0 |
| | Oxynex K flüssig | (Art.-Nr. 108324) | (1) | 0.05 |
| | | | | |
| B | Panthenol | (Art.-Nr. 501375) | (1) | 0.5 |
| | Karion F flüssig | (Art.-Nr. 102993) | (1) | 4.0 |
| | Konservierungsmittel | | | q.s. |
| | Wasser, demineralisiert | | | ad 100 |
| | | | | |
| C | Sepigel 305 | | (5) | 3.0 |
| | | | | |
| D | Ectoin | | (1) | 1.0 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Die vereinigte Phase B wird unter Rühren langsam in die Phase C eingetragen. Danach wird die vorgelöste Phase A zugesetzt. Es wird gerührt bis die Phasen homogen gemischt sind. Anschließend wird Phase D zugegeben und bis zur Homogenität gerührt.

### Bezugsquellen:

(1) Merck KGaA, Darmstadt
(2) Gustav Heess, Stuttgart
(3) Henkel KGaA, Düsseldorf
(4) BASF AG, Ludwigshafen
(5) Seppic, Frankreich

### Beispiel 2

Aus folgenden Komponenten wird eine erfindungsgemäße Hautpflegecreme (O/W) enthaltend Ectoin hergestellt:

| | | | | Gew.-% |
|---|---|---|---|---|
| A | Paraffin, dünnflüssig | (Art.-Nr. 107174) | (1) | 8.0 |
| | Isopropylmyristat | (Art.-Nr. 822102) | (1) | 4.0 |
| | Mirasil CM 5 | | (2) | 3.0 |
| | Stearinsäure | | (1) | 3.0 |
| | Arlacel 165 | | (3) | 5.0 |
| | | | | |
| B | Glycerin, 87 % | (Art.-Nr. 104091) | (1) | 3.0 |
| | Germaben II | | (4) | 0.5 |
| | Wasser, demineralisiert | | | ad 100 |
| | | | | |
| C | Parfüm Bianca | | (5) | 0.3 |
| | | | | |
| D | Ectoin | | (1) | 1.0 |

### Herstellung:

Zunächst werden die Phasen A und B getrennt auf 75 °C erwärmt. Danach wird Phase A unter Rühren langsam zu Phase B gegeben und solange gerührt bis eine homogene Mischung entsteht. Nach Homogenisierung der Emulsion wird unter Rühren auf 30 °C abgekühlt, die Phasen C und D zugegeben und bis zur Homogenität gerührt.

### Bezugsquellen:

(1) Merck KGaA, Darmstadt
(2) Rhodia
(3) ICI
(4) ISP
(5) Dragoco

### Beispiel 3

Aus folgenden Komponenten wird eine erfindungsgemäße Sonnenschutzlotion (W/O) enthaltend Ectoin hergestellt:

| | | | | Gew.-% |
|---|---|---|---|---|
| A | Abil WE 09 | | (2) | 5.0 |
| | Jojoba Öl | | (3) | 6.0 |
| | Cetiol V | | (4) | 6.0 |
| | Prisorine 2021 | | (5) | 4.5 |
| | Ricinusöl | | (6) | 1.0 |
| | Lunacera M | | (7) | 1.8 |
| | Miglyol 812 Neutralöl | | (8) | 4.5 |
| | | | | |
| B | Eusolex T-2000 | (Art.-Nr. 105373) | (1) | 3.0 |
| | Glycerin, 87 % | (Art.-Nr. 104091) | (1) | 2.0 |
| | Natriumchlorid | (Art.-Nr. 106400) | (1) | 0.4 |
| | Konservierungsmittel | | | q.s. |
| | Wasser, demineralisiert | | | ad 100 |
| | | | | |
| C | Parfüm | | (5) | 0.3 |
| | | | | |
| D | Ectoin | | (1) | 1.0 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Zunächst wird Eusolex T-2000 in Phase B eingerührt und auf 80 °C erwärmt. Danach wird Phase A auf 75 °C erwärmt und unter Rühren Phase B langsam zugegeben. Es wird bis zur Homogenität gerührt und anschließend unter Rühren auf 30 °C abgekühlt. Danach werden die Phasen C und D zugegeben und bis zur Homogenität gerührt.

Bezugsquellen:
(1) Merck KGaA, Darmstadt
(2) Th. Goldschmidt AG, Essen
(3) H. Lamotte, Bremen
(4) Henkel KGaA, Düsseldorf
(5) Unichema, Emmerich
(6) Gustav Heess, Stuttgart
(7) H.B. Fuller, Lüneburg
(8) Hüls Troisdorf AG, Witten

### Beispiel 4

Aus folgenden Komponenten wird eine Hautpflegecreme (O/W) enthaltend Ectoin hergestellt:

| | | | | Gew.-% |
|---|---|---|---|---|
| A | Paraffin, dünnflüssig | (Art.-Nr. 107174) | (1) | 8.0 |
| | Isopropylmyristat | (Art.-Nr. 822102) | (1) | 4.0 |
| | Mirasil CM 5 | | (2) | 3.0 |
| | Stearinsäure | | (1) | 3.0 |
| | Arlacel 165 V | | (3) | 5.0 |
| | | | | |
| B | Glycerin, 87 % | (Art.-Nr. 104091) | (1) | 3.0 |
| | Germaben II | | (4) | 0.5 |
| | Wasser, demineralisiert | | | ad 100 |
| | | | | |
| D | Ectoin | | (1) | x |
| | | | | |
| x = 0 (Placebo), 2, 5 Gew.-% | | | | |

### Herstellung:

Zunächst werden die Phasen A und B getrennt auf 75 °C erwärmt. Danach wird Phase A unter Rühren langsam zu Phase B gegeben und solange gerührt bis eine homogene Mischung entsteht. Nach Homogenisierung der Emulsion wird unter Rühren auf 30 °C abgekühlt, Phase D zugegeben und bis zur Homogenität gerührt.

### Bezugsquellen:

(1) Merck KGaA, Darmstadt
(2) Rhodia
(3) ICI
(4) ISP

### Beispiel 4a

Mit den in Beispiel 4 beschriebenen Hautpflegecremes (O/W) enthaltend Ectoin wird eine in vivo Bestimmung des Transepidermalen Wasserverlustes (TEWL) nach Schädigung der Hautbarriere durch SDS-Behandlung durchgeführt. Zunächst wird die Haut der Probanden (N=5) am Unterarm eine Woche lang zweimal täglich mit der O/W-Emulsion (2 mg/cm²) enthaltend 2 % und 5 % Ectoin und einer Emulsion ohne Ectoin (Placebo) behandelt. Um den TEWL künstlich durch eine Schädigung der Hornbarriere zu erhöhen, wird die Haut anschließend mit 80 µl Natriumdodecylsulfat (SDS; 2% in Wasser) in einer Aluminiumkammer unter Okklusion über 24 h behandelt. Die TEWL-Bestimmung wird in einem Klimaraum bei 22 °C und einer Luftfeuchtigkeit von 60 % mit TEWAmeter TM210 durchgeführt. In Abb. 1 ist der TEWL vor und nach der Behandlung mit den Ectoin-haltigen Emulsionen, sowie nach der Schädigung der Hautbarriere durch SDS dargestellt. Die Werte zu Abb. 1 sind in Tab. 1 aufgeführt.

**Tab. 1**

| In vivo Bestimmung des Transepidermalen Wasserverlusts (TEWL) nach Schädigung der Hautbarriere durch SDS-Behandlung. | | | |
|---|---|---|---|
| | TEWL [g/m²/h] | | |
| | vor Behandlung | nach 1 Woche Ectoin-Behandlung | nach SDS-Stress |
| unbehandelt | 5,2 | 5,6 | 18 |
| Placebo | 5,2 | 5,8 | 15,1 |
| 2% Ectoin | 5,6 | 5,6 | 14,2 |
| 5% Ectoin | 5,1 | 5,6 | 10,8 |

### Beispiel 4b

Mit den in Beispiel 4 beschriebenen Hautpflegecremes (O/W) enthaltend Ectoin wird eine in vivo Bestimmung der Hautfeuchtigkeit nach Ectoin-Behandlung und Dehydratisierung mittels Kieselgel durchgeführt. Zunächst wird die Haut der Probanden (N=5) am Unterarm eine Woche lang zweimal täglich mit einer kosmetischen Formulierung (2 mg/cm²) enthaltend 2 % und 5 % Ectoin und einer Formulierung ohne Ectoin (Placebo) behandelt. Der Feuchtigkeitsgehalt der Haut wird vor dem Auftragen und nach 1 Woche vier Stunden nach dem letzten Auftragen bestimmt. Dann wird Kieselgel 60 (0,2 g/cm²) auf die Testareale des Unterarmes für zwei Stunden unter Okklusion aufgetragen (Dehydratisierung). Nach Entfernen des Kieselgels wird die Hautfeuchtigkeit nach 10 min., 2 h, 4 h und 24 h in einem Klimaraum bei 22 °C und einer Luftfeuchtigkeit von 60 % gemessen. Die Ergebnisse sind in Abb. 2 dargestellt.

### Beispiel 5

Mit einer wäßrigen Ectoin-Lösung gepuffert in PBS-Puffer (22,2 mmol/l Dinatriumhydrogenphosphat, 5,6 mmol/l Kaliumdihydrogenphosphat, 123,3 mmol/l Natriumchlorid und 10 mmol/l Glucose) wird eine Bestimmung der membranstabilisierenden Wirkung von Ectoin vorbehandelten Human-Erythrozyten gegenüber SDS durchgeführt. Hierzu wird der RBC-Test verwendet. Es wird die prozentuale Membranstabilisierung von mit Ectoin vorbehandelten Zellen bestimmt.

Human-Erythrozyten (2x10⁸ Zellen/ml) werden 1 Stunde mit 0 %, 0,1 %, 0,5 %, 1 % und 5 % Ectoin behandelt. Dann werden die Zellen 10 min. mit 0 bis 0,04 % SDS-Lösung gestresst. Anschließend wird spektrophotometrisch anhand des freien Hämoglobingehalts bestimmt wieviel Zellen lysiert worden sind. In Abb. 3 ist der prozentuale Unterschied der lysierten Zellen in Abhängigkeit von der Ectoinkonzentration aus der Vorbehandlung gegenüber einer unbehandelten Kontrolle gezeigt. Der Versuch wird N = 5 mal durchgeführt.

Zusätzlich werden Human-Erythrozyten (2x10⁸ Zellen/ml) 0 (Kontrolle), 6, 18 und 24 Stunden mit 1 % Ectoin behandelt. Dann werden die Zellen 10 min. mit 0 bis 0,04 % SDS-Lösung gestresst. Anschließend wird spektrophotometrisch anhand des freien Hämoglobingehalts bestimmt wieviel Zellen lysiert worden sind. In Abb. 4 ist der prozentuale Unterschied der lysierten Zellen in Abhängigkeit von der Ectoinkonzentration aus der Vorbehandlung gegenüber einer unbehandelten Kontrolle gezeigt. Der Versuch wird N = 5 mal durchgeführt.

## Patentansprüche

1. Verwendung von einer oder mehreren Verbindungen ausgewählt aus den Verbindungen der Formeln Ia und Ib 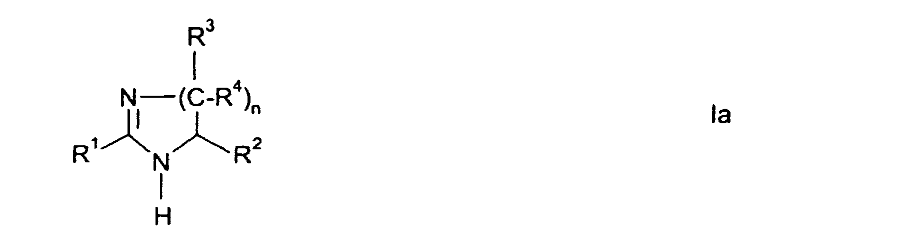 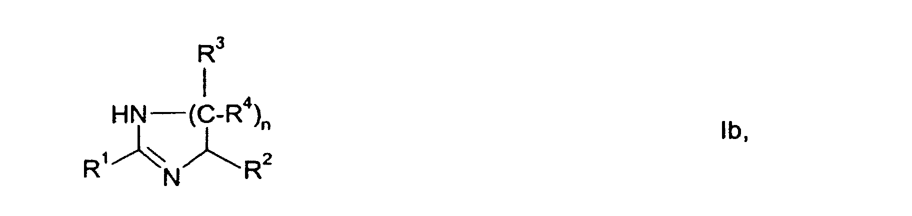 den physiologisch verträglichen Salzen der Verbindungen der Formeln la und Ib, und den stereoisomeren Formen der Verbindungen der Formeln la und Ib, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten,
zum Schutz von Zellen, Proteinen, und/oder Biomembranen der menschlichen Haut gegen die schädigende Wirkung von Tensiden, wobi es sich nicht um eine therapeutische Verwendung im Sinne des Art. 52 (4) EPÜ handelt.

2. Verwendung von einer oder mehreren Verbindungen ausgewählt aus den Verbindungen der Formeln la und Ib, den physiologisch verträglichen Salzen der Verbindungen der Formeln Ia und Ib und den stereoisomeren Formen der Verbindungen der Formeln la und Ib nach Anspruch 1 zum Schutz der Mikroflora der menschlichen Haut gegen die schädigende Wirkung von Tensiden, wobei es sich nicht um eine therapeutische Verwendung im Sinne des Art. 52 (4) EPÜ handelt.

3. Verwendung von einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib nach Anspruch 1 zur Stabilisierung der Hautbarriere gegen die schädigende Wirkung von Tensiden, wobei es sich nicht um eine therapeutische Verwendung im Sinne des Art. 52 (4) EPÜ handelt..

4. Verwendung nach einem der vorstehenden Ansprüche, **dadurch gekennzeichnet, daß** man eine oder mehrere Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib zur äußeren Anwendung in Form einer Lösung, einer Suspension, einer Emulsion, einer Paste, einer Salbe, eines Gels, einer Creme, einer Lotion, eines Puders, einer Seife, eines tensidhaltigen Reinigungspräparates, eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- und After-Sun-Präparats oder eines Sprays einsetzt.

5. Verwendung nach einem der vorstehenden Ansprüche, **dadurch gekennzeichnet, daß** der Anteil der Verbindungen ausgewählt aus den Verbindungen der Formeln Ia und Ib, den physiologisch verträglichen Salzen der Verbindungen der Formeln Ia und Ib, und den stereoisomeren Formen der Verbindungen der Formeln la und Ib von 0,0001 bis 50 Gew.% bezogen auf die gesamte kosmetische Formulierung beträgt.

6. Verwendung nach einem der vorstehenden Ansprüche, **dadurch gekennzeichnet, daß** die Verbindungen der Formeln Ia und Ib ausgewählt sind aus den Verbindungen (S)-1,4,5,6-Tetrahydro-2-methyl-4-pyrimidincarbonsäure und (S,S)-1,4,5,6-Tetrahydro-5-hydroxy-2-methyl-4-pyrimidincarbonsäure.

## Claims

1. Use of one or more compounds chosen from compounds of the formulae Ia and Ib 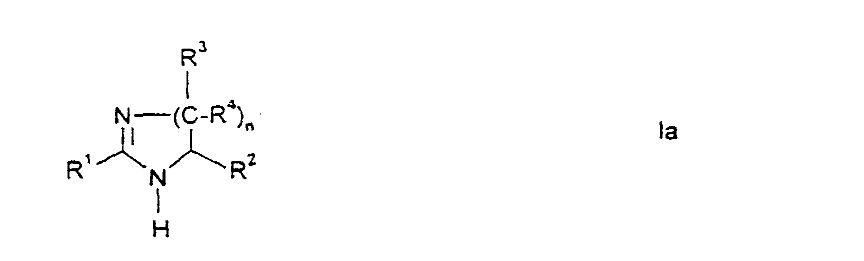 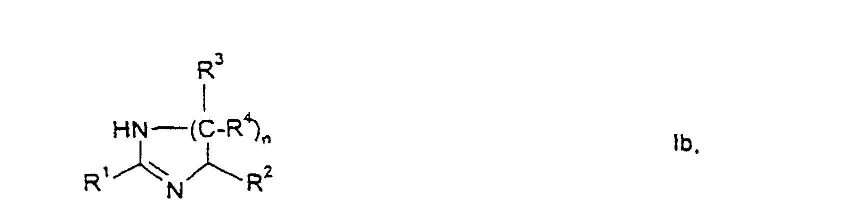 the physiologically compatible salts of the compounds of the formulae Ia and Ib, and the stereoisomeric forms of the compounds of the formulae Ia and Ib, where
R¹ is H or alkyl,
R² is H, COOH, COO-alkyl or CO-NH-R⁵,
R³ and R⁴ are in each case independently of one another H or OH,
n is 1, 2 or 3,
alkyl is an alkyl radical having 1 to 4 carbon atoms, and
R⁵ is H, alkyl, an amino acid radical, dipeptide radical or tripeptide radical
for the protection of cells, proteins and/or biomembranes in the human skin against the harmful effect of surfactants, said use not being a therapeutic use within the meaning of Article 52 (4) EPC.

2. Use of one or more compounds chosen from the compounds of the formulae Ia and Ib, the physiologically compatible salts of the compounds of the formulae Ia and Ib and the stereoisomeric forms of the compounds of the formulae Ia and Ib according to Claim 1 for the protection of the microflora of the human skin against the harmful effect of surfactants, said use not being a therapeutic use within the meaning of Article 52 (4) EPC.

3. Use of one or more compounds chosen from the compounds of the formulae Ia and Ib, the physiologically compatible salts of the compounds of the formulae Ia and Ib and the stereoisomeric forms of the compounds of the formulae Ia and Ib according to Claim 1 for stabilizing the skin barrier against the harmful effect of surfactants, said use not being a therapeutic use within the meaning of Article 52 (4) EPC.

4. Use according to one of the preceding claims, **characterized in that** one or more compounds chosen from the compounds of the formulae Ia and Ib, the physiologically compatible salts of the compounds of the formulae Ia and Ib and the stereoisomeric forms of the compounds of the formulae Ia and Ib are used for external application in the form of a solution, a suspension, an emulsion, a paste, an ointment, a gel, a cream, a lotion, a powder, a soap, a surfactant-containing cleansing preparation, an oil, a lipstick, a lipcare stick, a mascara, an eyeliner, eyeshadows, blusher, a powder, emulsion or wax foundation, a sunscreen, presun and aftersun preparation or a spray.

5. Use according to one of the preceding claims, **characterized in that** the proportion of the compounds chosen from the compounds of the formulae Ia and Ib, the physiologically compatible salts of the compounds of the formulae Ia and Ib, and the stereoisomeric forms of the compounds of the formulae Ia and Ib is from 0.0001 to 50% by weight, based on the total cosmetic formulation.

6. Use according to one of the preceding claims, **characterized in that** the compounds of the formulae Ia and Ib are chosen from the compounds (S)-1,4,5,6-tetrahydro-2-methyl-4-pyrimidinecarboxylic acid and (S,S)-1,4,5,6-tetrahydro-5-hydroxy-2-methyl-4-pyrimidinecarboxylic acid.

## Revendications

1. Utilisation d'un ou plusieurs composés choisis parmi les composés de formules Ia et Ib 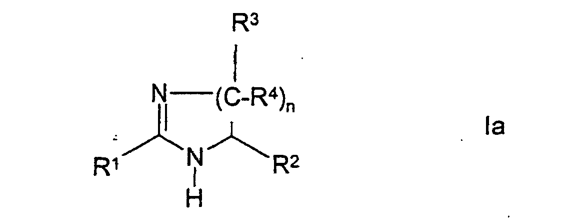 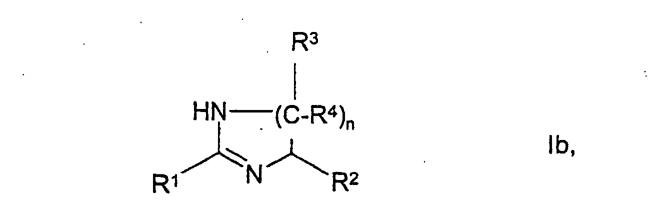 des sels physiologiquement acceptables des composés de formules Ia et Ib et des formes stéréoisomères des composés de formules Ia et Ib,
R¹ représentant H ou un alkyle,
R² H, COOH, COO-alkyle ou CO-NH-R⁵,
R³ et R⁴ représentant chacun indépendamment l'un de l'autre, H ou OH,
n étant égal à 1, 2 ou 3,
alkyle représentant un reste alkyle comportant 1 à 4 atomes de carbone, et
R⁵ H, un alkyle, un reste acide aminé, un reste dipeptide ou un reste tripeptide,
pour la protection des cellules, protéines, et/ou biomembranes de la peau humaine contre l'action nuisible des agents tensioactifs, ladite utilisation n'étant pas une utilisation thérapeutique au sens de l'art. 52(4) de la CBE.

2. Utilisation d'un ou plusieurs composés choisis parmi les composés de formules Ia et Ib, les sels physiologiquement acceptables des composés de formules Ia et Ib et les formes stéréoisomères des composés de formules Ia et Ib selon la revendication 1 pour la protection de la microflore de la peau humaine contre l'action nuisible des agents tensioactifs, ladite utilisation n'étant pas une utilisation thérapeutique au sens de l'art. 52(4) de la CBE.

3. Utilisation d'un ou plusieurs composés choisis parmi les composés de formules Ia et Ib, les sels physiologiquement acceptables des composés de formules Ia et Ib et les formes stéréoisomères des composés de formules Ia et Ib selon la revendication 1 pour la stabilisation de la barrière cutanée contre l'action nuisible des agents tensioactifs, ladite utilisation n'étant pas une utilisation thérapeutique au sens de l'art. 52(4) de la CBE.

4. Utilisation selon l'une des revendications précédentes, **caractérisée en ce que** l'on utilise un ou plusieurs composés choisis parmi les composés de formules Ia et Ib, les sels physiologiquement acceptables des composés de formules Ia et Ib et les formes stéréoisomères des composés de formules Ia et Ib pour un usage externe sous forme d'une solution, d'une suspension, d'une émulsion, d'une pâte, d'un onguent, d'un gel, d'une crème, d'une lotion, d'une poudre, d'un savon, d'un produit nettoyant contenant des agents tensioactifs, d'une huile, d'un rouge à lèvres, d'un stick de soins pour lèvres, d'un mascara, d'un eyeliner, d'une ombre pour paupière, de rouge, d'un maquillage sous forme de poudre, d'émulsion ou de cire, d'une préparation pour la protection solaire et de préparations avant-soleil et après-soleil ou d'un spray.

5. Utilisation selon l'une des revendications précédentes, **caractérisée en ce que** la proportion des composés choisis parmi les composés de formules la et Ib, les sels physiologiquement acceptables des composés de formules Ia et Ib et les formes stéréoisomères des composés de formules Ia et Ib est comprise entre 0,0001 et 50 % en poids rapportée à la totalité de la formulation cosmétique.

6. Utilisation selon l'une des revendications précédentes, **caractérisée en ce que** les composés de formules Ia et Ib sont choisis parmi les composés acide (S)-1,4,5,6-tétrahydro-2-méthyl-4-pyrimidine-carboxyllque et acide (S,S)-1,4,5,6-tétrahydro-5-hydroxy-2-méthyl-4-pyrimidine-carboxylique.
